# FASCICULE DE BREVET EUROPEEN

(11) **EP 2 198 929 B1**
(45) Date de publication et mention de la délivrance du brevet: **06.03.2019**
(21) Numéro de dépôt: 09180003.7
(22) Date de dépôt: 18.12.2009
(51) Int. Cl.: A61Q 5/10, A61K 8/34, A61K 8/41, A61K 8/31, A61K 8/81, A61K 8/84

(54) **Composition comprenant un corps gras et un polymere cationique, procede de coloration ou d'eclaircissement la mettant en oeuvre et dispositifs**
Zusammensetzung enthaltend einen Fettkörper und einen kationisches Polymere und deren Verwendung als Haarfärbemittel oder Aufheller
Hair dye or lightening composition comprising a fatty substance and a cationic polymer, and use thereof

(30) Priorité: 19.12.2008 FR 0807310
(43) Date de publication de la demande: 23.06.2010
(73) Titulaire: L'Oréal, 75008 Paris (FR)
(72) Inventeur: Hercouet, Leïla, 93360, Neuilly Plaisance (FR); Simonet, Frédéric, 92110, Clichy (FR); Audousset, Marie-Pascale, 92600, Asnieres (FR); Rapold, Philippe, 75015, Paris (FR)
(74) Mandataire: Wattremez, Catherine

(56) Documents cités:
- EP-A- 1 449 512
- EP-A- 1 792 602
- WO-A-98/03150
- WO-A-2005/055966
- DE-A1-102006 012 575
- JP-A- 1 165 514
- US-A1- 2006 260 071
- US-B1- 6 238 653

## Description

La présente invention a pour objet une composition de coloration des fibres kératiniques humaines telle que revendiquée dans la présente revendication 1, comprenant, outre les colorants d'oxydation et l'agent oxydant, une teneur élevée en corps gras et un polymère cationique. L'invention concerne également un procédé de coloration la mettant en oeuvre.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

En général, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Il est également possible d'ajouter à ces compositions, des colorants directs, qui sont des molécules colorées et colorantes ayant une affinité pour les fibres. Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. La présence de tels composés permet d'enrichir encore la coloration obtenue, en reflets ou bien d'augmenter la chromaticité de la coloration obtenue.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

La coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

Les compositions obtenues doivent, en outre, présenter de bonnes propriétés de mélange et d'application, et notamment de bonnes propriétés rhéologiques pour ne pas couler, lors de leur application, sur le visage, le cuir chevelu, ou en dehors des zones que l'on se propose de teindre.

De nombreuses tentatives ont été faites dans le domaine de la coloration capillaire afin d'améliorer les propriétés tinctoriales, à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et aux propriétés d'application de la coloration. Le document JP2003/81790 décrit un procédé de coloration d'oxydation mettant en oeuvre une composition comprenant un colorant d'oxydation et un agent alcalin et une composition oxydante comprenant des corps gras dont au moins un monoéther de glycerine.

En ce qui concerne les procédés d'éclaircissement des fibres kératiniques, on emploie des compositions aqueuses comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres.

L'un des buts de la présente invention est d'obtenir des compositions pour la coloration, en particulier pour la coloration d'oxydation des fibres kératiniques qui ne présentent pas les inconvénients de l'art antérieur.

Plus particulièrement, l'un des buts de la présente invention est d'obtenir des compositions de coloration, en particulier d'oxydation, des fibres kératiniques, présentant des propriétés tinctoriales améliorées qui permettent d'atteindre l'éclaircissement souhaité et qui soient faciles à mélanger et à appliquer, notamment qui ne coulent pas et restent bien localisées au point d'application. Par propriétés tinctoriales améliorées, on entend en particulier une amélioration au niveau de la puissance/intensité et/ou de l'homogénéité de la teinture.

Ces buts et d'autres sont atteints par la présente invention qui a donc pour objet une composition de coloration de fibres kératiniques humaines, caractérisée en ce qu'elle comprend, dans un milieu cosmétiquement acceptable :
(a) au moins 25 % en poids d'un ou plusieurs corps gras ;
(b) un ou plusieurs polymères cationiques ;
(c) un ou plusieurs colorants choisis parmi les colorants d'oxydation, les colorants directs, ou leurs mélanges ; un ou plusieurs agents alcalinisants ; ou leurs mélanges ;
(d) un ou plusieurs agents oxydants.

La présente invention a pour objet une composition de coloration de fibres kératiniques humaines, caractérisée en ce qu'elle comprend, dans un milieu cosmétiquement acceptable:
(a) au moins 25 % en poids d'un ou plusieurs corps gras ;
(b) un ou plusieurs polymères cationiques ;
(c) un ou plusieurs colorants d'oxydation ;
(d) un ou plusieurs agents oxydants et
(e) éventuellement un ou plusieurs agents alcalins.

Elle concerne également un procédé de coloration des fibres kératiniques humaines consistant à mettre en oeuvre la composition précitée.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, la composition de coloration selon l'invention comprend au moins 25 % en poids d'un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins une chaîne hydrocarbonée comportant au moins 6 atomes de carbone ou un enchaînement d'au moins deux groupements siloxane. En outre, les corps gras sont généralement solubles dans des solvants organiques dans les mêmes conditions de température et de pression, comme par exemple le chloroforme, l'éthanol le benzène, l'huile de vaseline ou le décaméthylcyclopentasiloxane.

Selon l'invention, les corps gras sont choisis parmi les composés liquides ou pâteux à température ambiante et à pression atmosphérique.

Plus particulièrement, le ou les corps gras sont choisis parmi les alcanes inférieurs en C₆-C₁₆, les huiles non siliconées d'origine animale, végétale minérale ou synthétique, les alcools gras, les acides gras, les esters d'acide gras et/ou d'alcool gras, les cires non siliconées, les silicones.

Il est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires, ramifiés, éventuellement cycliques. A titre d'exemple, on peut citer l'hexane, le dodécane, les isoparaffines comme l'isohexadécane, l'isodécane.

Comme huiles d'origine animale, végétale, minérale ou synthétique, utilisables dans la composition de l'invention, on peut citer par exemple :
- les huiles hydrocarbonées d'origine animale, telles que le perhydrosqualène.
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité.
- les hydrocarbures linéaires ou ramifiés, d'origine minérale ou synthétique, de plus de 16 atomes de carbone, tels que les huiles de paraffine, volatiles ou non, et leurs dérivés, la vaseline, l'huile de vaseline, les polydécènes, le polyisobutène hydrogéné tel que Parléam®.
- les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les acides gras utilisables dans le cadre de l'invention, sont plus particulièrement choisis parmi les acides carboxyliques, saturés ou insaturés, comportant de 6 à 30 atomes de carbone, en particulier de 9 à 30 atomes de carbone. Ils sont avantageusement choisis parmi l'acide myristique, l'acide palmitique, l'acide stéarique, l'acide béhénique, l'acide oléïque, l'acide linoléïque, l'acide linolénique et l'acide isostéarique,

En ce qui concerne les esters d'acide gras et/ou d'alcools gras, avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de de préférence à 1% encore plus préférentiellement à 0,1%). Ils présentent dans leur structure au moins une chaîne hydrocarbonée comportant au moins 6 atomes de carbone ou un enchaînement d'au moins deux groupements siloxane. En outre, les corps gras sont généralement solubles dans des solvants organiques dans les mêmes conditions de température et de pression, comme par exemple le chloroforme, l'éthanol le benzène, l'huile de vaseline ou le décaméthylcyclopentasiloxane.

Selon l'invention, les corps gras sont choisis parmi les composés liquides ou pâteux à température ambiante et à pression atmosphérique.

Plus particulièrement, le ou les corps gras sont choisis parmi les alcanes inférieurs en C₆-C₁₆, les huiles non siliconées d'origine animale, végétale minérale ou synthétique, les alcools gras, les acides gras, les esters d'acide gras et/ou d'alcool gras, les cires non siliconées, les silicones.

Il est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires, ramifiés, éventuellement cycliques. A titre d'exemple, on peut citer l'hexane, le dodécane, les isoparaffines comme l'isohexadécane, l'isodécane.

Comme huiles d'origine animale, végétale, minérale ou synthétique, utilisables dans la composition de l'invention, on peut citer par exemple :
- les huiles hydrocarbonées d'origine animale, telles que le perhydrosqualène.
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité.
- les hydrocarbures linéaires ou ramifiés, d'origine minérale ou synthétique, de plus de 16 atomes de carbone, tels que les huiles de paraffine, volatiles ou non, et leurs dérivés, la vaseline, l'huile de vaseline, les polydécènes, le polyisobutène hydrogéné tel que Parléam®.
- les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les acides gras utilisables dans le cadre de l'invention, sont plus particulièrement choisis parmi les acides carboxyliques, saturés ou insaturés, comportant de 6 à 30 atomes de carbone, en particulier de 9 à 30 atomes de carbone. Ils sont avantageusement choisis parmi l'acide myristique, l'acide palmitique, l'acide stéarique, l'acide béhénique, l'acide oléïque, l'acide linoléïque, l'acide linolénique et l'acide isostéarique,

En ce qui concerne les esters d'acide gras et/ou d'alcools gras, avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou dioléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination Ryoto Sugar Esters par exemple référencés B370 et correspondant au béhénate de saccharose formé de 20% de monoester et 80% de di-triester-polyester;
- le mono-di-palmito-stéarate de sucrose commercialisé par la société Goldschmidt sous la dénomination Tegosoft® PSE.

La cire ou les cires non siliconées sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 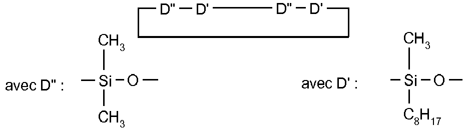
   On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le ou les corps gras ne comprennent pas de motif oxyalkyléné en C₂-C₃ ni de motif glycérolé.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Les corps gras sont de préférence choisis parmi les alcanes inférieurs en C₆-C₁₆, les huiles non siliconées d'origine végétale, minérales ou synthétique, les alcools gras, les esters d'acide gras et/ou d'alcool gras, les silicones, ou leurs mélanges.

De préférence, le corps gras est choisi parmi l'huile de vaseline, les polydécènes, les esters d'acide gras et/ou d'alcool gras liquides, ou leurs mélanges.

Comme indiqué auparavant, la composition selon l'invention comprend au moins 25% de corps gras. La composition selon l'invention présente plus particulièrement une teneur en corps gras allant de 25 à 80% en poids, encore plus préférentiellement de 25 à 65%, mieux de 30 à 55% en poids par rapport au poids de la composition.

La composition selon l'invention comprend par ailleurs un ou plusieurs polymères cationiques.

De préférence, les polymères cationiques entrant dans la composition de l'invention ne sont pas choisis parmi les polymères associatifs cationiques. En d'autres termes, ces polymères cationiques ne comprennent pas dans leur structure de chaîne hydrophobe pendante ou terminale, notamment de type alkyle ou alcényle, comprenant de 10 à 30 atomes de carbone.

Les polymères cationiques de la composition selon l'invention sont de manière avantageuse, choisis parmi les polymères suivants, ainsi que leurs mélanges :
**(1)** Les homopolymères ou copolymères dérivés d'esters ou d'amides acryliques ou méthacryliques et comportant au moins un des motifs de formules (I), (II), (III) ou (IV) suivantes: 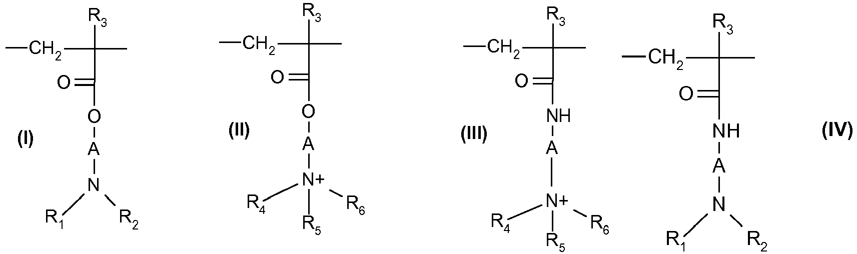 dans lesquelles:
   R₃, identiques ou différents, désignent un atome d'hydrogène ou un radical CH₃;
   A, identiques ou différents, représentent un groupe alkyle, linéaire ou ramifié, en C₁-C₆, de préférence en C₂-C₃ ou un groupe hydroxyalkyle en C₁-C₄ ;
   R₄, R₅, R₆, identiques ou différents, représentent un groupe alkyle en C₁-C₁₈ ou un radical benzyle, et de préférence un groupe alkyle en C₁-C₆ ;
   R₁ et R₂, identiques ou différents, représentent hydrogène ou un groupe alkyle en C₁-C₆, et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthyl-amino-éthyl méthacrylate quaternisé au sulfate de diméthyle ou avec un halogénure de diméthyle, tel que celui vendu sous la dénomination Hercofloc par la société Hercules,
   - les copolymères d'acrylamide et de chlorure de méthacryloyloxy-éthyl-triméthyl-ammonium décrits par exemple dans EP 80976 et vendus sous la dénomination Bina Quat P 100 par la société Ciba Geigy,
   - le copolymère d'acrylamide et de méthosulfate de méthacryloyloxy-éthyl-triméthyl-ammonium vendu sous la dénomination Reten par la société Hercules,
   - les copolymères vinylpyrrolidone / acrylate ou méthacrylate de dialkylaminoalkyle quaternisés ou non, tels que les produits vendus sous la dénomination "Gafquat" par la société ISP comme par exemple "Gafquat 734" ou "Gafquat 755" ou bien les produits dénommés "Copolymer 845, 958 et 937". Ces polymères sont décrits dans FR 2077143 et FR 2393573,
   - les terpolymères méthacrylate de diméthyl amino éthyle/vinylcaprolactame/ vinylpyrrolidone tel que le produit vendu sous la dénomination Gaffix VC 713 par la société ISP,
   - les copolymère vinylpyrrolidone/méthacrylamidopropyl dimethylamine commercialisés notamment sous la dénomination Styleze CC 10 par ISP,
   - les copolymères vinylpyrrolidone / méthacrylamide de diméthyl-amino-propyle quaternisés tel que le produit vendu sous la dénomination "Gafquat HS 100" par la société ISP.
   - et les polymères réticulés de sels de méthacryloyloxyalkyl(C₁-C₄) trialkyl(C₁-C₄) ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "Salcare® SC 92" par la Société Ciba. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de "Salcare® SC 95 " et "Salcare® SC 96" par la Société Ciba.
**(2)** Les dérivés d'éthers de cellulose comportant des groupements ammonium quaternaire décrits dans FR1492597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les copolymères de cellulose ou les dérivés de cellulose greffés avec un monomère hydrosoluble d'ammonium quaternaire, et décrits notamment dans US 4131576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthyl-ammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les polysaccharides cationiques non cellulosiques notamment décrits dans les brevets US3589578 et 4031307 tels que les gommes de guar contenant des groupements cationiques trialkylammonium. On utilise par exemple des gommes de guar modifiées par un sel (par exemple chlorure) de 2,3-époxypropyl triméthylammonium.
   De tels produits sont commercialisés notamment sous les dénominations commerciales de Jaguar C13S, Jaguar C15, Jaguar C17 ou Jaguar C162 par la société Meyhall.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR2162025 et FR2280361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans FR2252840 et FR2368508 ;
   Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylamino-hydroxyalcoyldialoylène triamine dans lesquels le radical alcoyle est en C₁-C₄ et désigne de préférence méthyle, éthyle, propyle. De tels polymères sont notamment décrits dans FR1583363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(7)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C₃-C₈. Le rapport molaire entre le polyalkylène polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits US 3227615 et US 2961347.
   Des polymères de ce type sont en particulier commercialisés sous la dénomination "Hercosett 57", "PD 170" ou "Delsette 101" par la société Hercules.
**(8)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 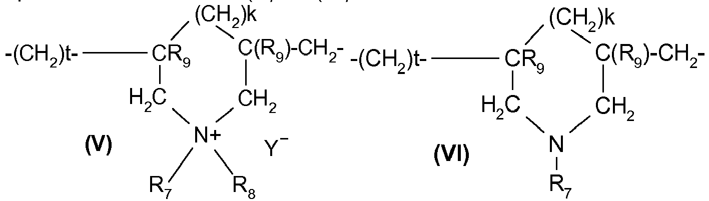 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle en C₁-C₈, un groupement hydroxyalkyle dans lequel le groupement alkyle est en C₁-C₅, un groupement amidoalkyle dont l'alkyle est en C₁-C₄ ; R₇ et R₈ peuvent encore désigner conjointement avec l'atome d'azote auquel ils sont rattachés, un groupement hétérocyclique, tels que pipéridinyle ou morpholinyle ; R₇ et R₈ indépendamment l'un de l'autre désignent de préférence un groupement alkyle en C₁-C₄ ; Y⁻ est un anion organique ou minéral tel que bromure, chlorure, acétate, borate, citrate, tartrate, bisulfate, bisulfite, sulfate, phosphate. Ces polymères sont notamment décrits dans FR2080759 et FR2190406.
   Parmi les polymères définis ci-dessus, on peut citer plus particulièrement l'homopolymère de chlorure de diméthyldiallylammonium vendu sous la dénomination "Merquat 100" par la société Nalco (et ses homologues de faible masse moléculaire moyenne en poids) et les copolymères de chlorure de diallyldiméthylammonium et d'acrylamide commercialisés sous la dénomination "Merquat 550".
**(9)** Les polymères de diammonium quaternaires contenant des motifs récurrents répondant à la formule : 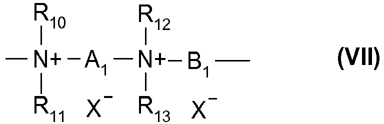 formule dans laquelle :
   R₁₀, R₁₁, R₁₂ et R₁₃, identiques ou différents, représentent des radicaux aliphatiques, alicycliques, ou arylaliphatiques en C₁-C₆ ou des radicaux hydroxyalkylaliphatiques dont le radical alkyle est en C₁-C₄, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A₁ et B₁ représentent des groupements polyméthyléniques en C₂-C₆ linéaires ou ramifiés, saturés ou non, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₀ et R₁₂ peuvent former avec les deux atomes d'azote auxquels ils sont rattachés un cycle pipérazinique ;
   en outre si A₁ désigne un radical alkylène ou hydroxyalkylène linéaire ou ramifié, saturé ou insaturé, B₁ peut également désigner un groupement -(CH₂)ₙ-CO-D-OC-(CH₂)ₙ- dans lequel n allant de 1 à 6 et D désigne un groupement ammonium quaternaire :
      a) un reste de glycol de formule : -O-Z-O-, où Z désigne un radical hydrocarboné linéaire ou ramifié ou un groupement répondant à l'une des formules suivantes : -(CH₂-CH₂-O)ₓ-CH₂-CH₂- ; -[CH₂-CH(CH₃)-O]_{y}-CH₂-CH(CH₃)- où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical -CH₂-CH₂-S-S-CH₂-CH₂- ;
      d) un groupement uréylène de formule : -NH-CO-NH-.
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans FR2320330, FR2270846, FR2316271, FR2336434, FR2413907, US2273780, US2375853, US2388614, US2454547, US3206462, US2261002, US2271378, US3874870, US4001432, US3929990, US3966904, US4005193, US4025617, US4025627, US4025653, US4026945 et US4027020.
   On peut utiliser plus particulièrement les polymères qui sont constitués de motifs récurrents répondant à la formule (VIII) suivante: 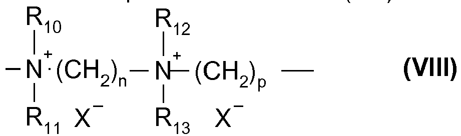 dans laquelle R₁₀, R₁₁, R₁₂ et R₁₃, identiques ou différents, désignent un radical alkyle ou hydroxyalkyle en C₁-C₄, n et p sont des nombres entiers variant de 2 à 6 environ et, X⁻ est un anion dérivé d'un acide minéral ou organique.
   De manière encore plus avantageuse, les polymères cationiques correspondant à cette famille comprennent les motifs récurrents de formules (W) et (U) suivantes : 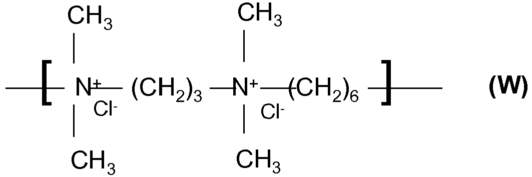 et notamment ceux dont le poids moléculaire, déterminé par chromatographie par perméation de gel, est compris entre 9500 et 9900; 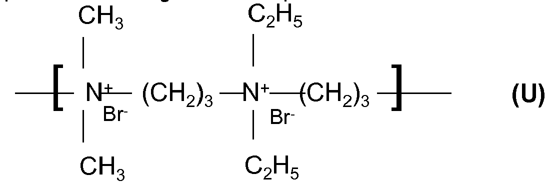 et notamment ceux dont le poids moléculaire, déterminé par chromatographie par perméation de gel, est d'environ 1200.
**(10)** Les polymères de polyammonium quaternaire constitués de motifs récurrents de formule (IX) : 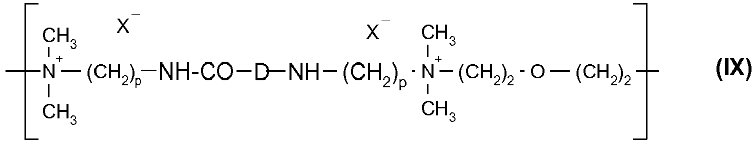 dans laquelle p désigne un nombre entier variant de 1 à 6 environ, D peut être nul ou peut représenter un groupement -(CH₂)ᵣ-CO- dans lequel r désigne un nombre allant de 1 à 6 environ, X⁻ est un anion.
   De tels polymères peuvent être préparés selon les procédés décrits dans US4157388, US4702906, US4719282. Ils sont notamment décrits dans la demande de brevet EP122324. Parmi eux, on peut par exemple citer, les produits "Mirapol A 15", "Mirapol AD1", "Mirapol AZ1" et "Mirapol 175" vendus par la société Miranol.
**(11)** Les polymères quaternaires de vinylpyrrolidone et de vinylimidazole tels que par exemple les produits commercialisés sous les dénominations Luviquat FC 905, FC 550 et FC 370 par la société BASF.
**(12)** Les homopolymères ou copolymères de vinylamide et en particulier les homopolymères de vinylamide partiellement hydrolysés tels que les poly(vinylamine/vinylamide).
**(13)** Les dérivés de polyuréthane cationique, de préférence ceux à caractère élastique formé de la réaction :
   (a1) d'au moins un motif cationique résultant d'au moins une amine tertiaire ou quaternaire présentant au moins deux fonctions réactives à hydrogène labile,
   (a2) d'au moins un mélange d'au moins deux motifs non ioniques différents présentant au moins deux fonctions réactives à hydrogène labile, de préférence choisies parmi les groupes hydroxyle, amine primaire ou amine secondaire, et les groupes thiol,
   (b) d'au moins un composé comportant au moins deux fonctions isocyanate,
**(14)** D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, et les dérivés de la chitine.

De préférence, le ou les polymères cationiques sont choisis parmi les polymères des familles (1), (2), (3), (4), (8), (9).

Plus particulièrement, la charge cationique des polymères utilisables dans le cadre de l'invention est d'au moins 1 meq/g, plus avantageusement d'au moins 2 meq/g. La densité de charge peut être déterminée selon la méthode Kjeldahl. Elle est mesurée en général à un pH de l'ordre de 3 à 9. Elle peut être aussi déterminée par calcul à partir de la structure du polymère.

Encore plus préférentiellement le polymère cationique présent dans la composition selon l'invention est un polymère des familles (8) et (9).

Mieux encore le polymère cationique présent dans la composition selon l'invention est l'homopolymère de chlorure de diméthyldiallylammonium et/ou un polymère de formule (U) ou (W)

La composition selon l'invention comprend avantageusement de 0,005 à 5 % en poids, plus particulièrement de 0,05 à 0,5% en poids de polymère(s) cationique(s) par rapport au poids de la composition.

La composition selon l'invention peut éventuellement comprendre un ou plusieurs colorants choisis parmi les colorants d'oxydation, les colorants directs ou leurs mélanges qui seront détaillés ci-dessous.

De préférence, la composition selon l'invention comprend un ou plusieurs colorants d'oxydation.

Les colorants d'oxydation sont en général choisis parmi une ou plusieurs bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB1026978 et GB1153196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a]pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE2359399 ; JP88-169571 ; JP05-63124 ; EP0770375 ou demande de brevet WO96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE3843892, DE4133957 et demandes de brevet WO94/08969, WO94/08970, FR-A-2733749 et DE19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10% en poids par rapport au poids total de la composition, et de préférence de 0,005 à 5% en poids par rapport au poids total de la composition.

La teneur en coupleur(s), s'il(s) est(sont) présent(s), représentent chacun avantageusement de 0,0001 à 10% en poids par rapport au poids total de la composition, et de préférence de 0,005 à 5% en poids par rapport au poids total de la composition.

La composition selon l'invention peut éventuellement comprendre, éventuellement en plus du ou des colorants d'oxydation et de préférence en plus du ou des colorants d'oxydation, un ou plusieurs colorants directs, synthétiques ou naturels, choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10% en poids du poids total de la composition, et de préférence de 0,005 à 5% en poids.

La composition selon l'invention comprend également un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, ainsi que les peracides et leurs précurseurs.

De préférence, l'agent oxydant n'est pas choisi parmi les sels peroxygénés. Avantageusement, l'agent oxydant est le peroxyde d'hydrogène.

La teneur en agent(s) oxydant(s) représente plus particulièrement de 0,1 à 20% en poids, de préférence de 0,5 à 10% en poids, par rapport au poids de la composition.

La composition selon l'invention peut également comprendre un ou plusieurs agents alcalinisants.

L'agent alcalinisant peut être minéral ou organique ou hybride.

Le ou les agents alcalinisants minéraux sont de préférence choisis parmi l'ammoniaque, les carbonates ou bicarbonates alcalins, les hydroxydes de sodium ou de potassium ou leurs mélanges.

Le ou les, agents alcalinisants organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

Le ou les agents alcalinisants organiques sont par exemple choisis parmi les alcanolamines, les éthylènediamines oxyéthylénées et/ou oxypropylénées, les acides aminés et les composés de formule (IX) suivante : 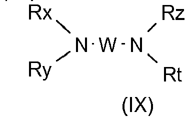 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Par alcanolamine on entend une amine organique comprenant une fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino-méthane.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (X) suivante : 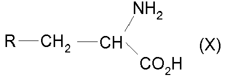 où R désigne un groupe choisi parmi :

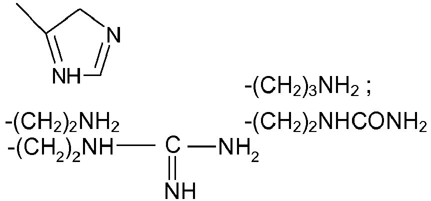

Les composés correspondants à la formule (X) sont l'histidine, la lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la baleine

L'amine organique peut aussi être choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique présente dans la composition de l'invention est une alcanolamine.

Encore plus préférentiellement l'amine organique est la monoéthanolamine.

A titre de composés hybrides on peut mentionner les sels des amines citées précédemment avec des acides comme l'acide carbonique, l'acide chlorhydrique.

On peut en particulier utiliser le carbonate de guanidine ou le chlorhydrate de monoéthanolamine.I

De manière avantageuse, la composition selon l'invention présente une teneur en agent(s) alcalinisant(s) allant de 0,01 à 30 % en poids, de préférence de 0,1 à 20 % en poids par rapport au poids de ladite composition.

Il est à noter que, de préférence, la composition selon l'invention ne comprend pas d'ammoniaque, en tant qu'agent alcalinisant. Si toutefois elle en comprenait, sa teneur ne dépasserait pas 0,03% en poids (exprimé en NH₃), de préférence ne dépasserait pas 0,01% en poids, par rapport au poids de la composition selon l'invention.

La composition de l'invention contient de préférence une ou plusieurs alcanolamines, et/ou un ou plusieurs acides aminés basiques.

De préférence la composition de l'invention comprend de la monoéthanolamine.

La composition selon l'invention peut également un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 100, de préférence entre 2 et 50 de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés comprenant de 1 à 100 moles d'oxyde d'éthylène ; les esters d'acides en C₈-C₃₀, saturés ou non, linéaires ou ramifiés, et de sorbitol polyoxyéthylénés comprenant de 1 à 100 moles d'oxyde d'éthylène.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition est un tensioactif non ionique.

La teneur en tensioactifs dans la composition représente plus particulièrement de 0,1 à 50% en poids, de préférence de 0,5 à 30 % en poids par rapport au poids de la composition.

La composition peut également renfermer divers adjuvants utilisés classiquement dans les compositions pour la coloration des cheveux, tels que des polymères anioniques, non ioniques, amphotères, zwitterioniques ou leurs mélanges ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20% en poids par rapport au poids de la composition.

La composition peut comprendre un ou plusieurs agents épaississants minéraux choisis parmi les argiles organophiles, les silices pyrogénées, ou leurs mélanges.

L'argile organophile peut être choisie parmi la montmorrillonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay ; les quaternium-18/benzalkonium bentonite telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel IPP, Bentone Gel ISD, Bentone Gel SS71, Bentone Gel VS8, Bentone Gel VS38 par la société Rhéox et Simagel M, Simagel SI 345 par la société Biophil.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, la composition comprend une hectorite, une bentonite organomodifiée ou une silice pyrogénée éventuellement modifiée.

Selon une variante, la composition selon l'invention est obtenue en mélangeant une première composition comprenant un ou plusieurs corps gras, un ou plusieurs colorants d'oxydation, avec une deuxième composition comprenant un ou plusieurs agents oxydants ; le ou les polymères cationiques se trouvant dans la première composition et/ou dans la deuxième composition et le ou les éventuels agents alcalinisants se trouvant également dans la première composition et/ou dans la deuxième composition et de préférence dans la première composition.

Selon une deuxième variante de l'invention, la composition selon l'invention est obtenue en mélangeant une première composition comprenant un ou plusieurs corps gras ; une deuxième composition comprenant comprenant un ou plusieurs colorants choisis parmi les colorants d'oxydation, les colorants directs, ou leurs mélanges ; un ou plusieurs agents alcalinisants ; ou leurs mélanges ; et une troisième composition comprenant un ou plusieurs agents oxydants ; le ou les polymères cationiques se trouvant dans l'une ou l'autre des trois compositions précitées. La première composition peut en particulier être anhydre.

Selon une variante additionnelle de l'invention, la composition selon l'invention est obtenue en mélangeant une première composition comprenant un ou plusieurs corps gras ; une deuxième composition comprenant un ou plusieurs colorants d'oxydation ; et une troisième composition comprenant un ou plusieurs agents oxydants ; le ou les polymères cationiques se trouvant dans l'une ou l'autre des trois compositions précitées et le ou les éventuels agents alcalinisants se trouvant également dans l'une ou l'autre des trois compositions précitées et de préférence dans la première et/ou la seconde composition. La première composition peut en particulier être anhydre.

Les ingrédients des compositions précitées et leurs teneurs sont déterminés en fonction des caractéristiques détaillées auparavant pour la composition finale selon l'invention.

Dans chacune des variantes précitées, la composition oxydante est de préférence une composition aqueuse. En particulier, elle comprend plus de 5% en poids d'eau, de préférence plus de 10% en poids d'eau, et de manière encore plus avantageuse plus de 20% en poids d'eau.

Elle peut également comprendre un ou plusieurs solvants organiques choisis parmi ceux listés auparavant ; ces derniers représentant plus particulièrement, lorsqu'ils sont présents, de 1 à 40% en poids par rapport au poids de la composition oxydante, et de préférence de 5 à 30% en poids.

La composition oxydante comprend également de manière préférée, un ou plusieurs agents acidifiants. Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante, lorsqu'elle est aqueuse, est inférieur à 7.

De préférence, la composition oxydante comprend du peroxyde d'hydrogène en tant qu'agent oxydant, en solution aqueuse, dont la concentration varie, plus particulièrement, de 0,1 à 50% en poids, de préférence entre 0,5 et 20% en poids, et encore plus préférentiellement entre 1 et 15% en poids, par rapport au poids de la composition oxydante.

Le procédé de coloration selon l'invention consiste donc à appliquer la composition selon l'invention, sur les fibres kératiniques humaines, sèches ou humides.

La composition est ensuite laissée en place pendant une durée allant habituellement d'une minute à une heure, de préférence de 5 minutes à 30 minutes.

La température durant le procédé est classiquement comprise entre la température ambiante (entre 15 à 25 °C) et 80 °C, de préférence entre la température ambiante et 60 °C.

A l'issue du traitement, les fibres kératiniques humaines sont éventuellement rincées à l'eau, subissent éventuellement un lavage avec un shampooing suivi d'un rinçage à l'eau, avant d'être séchées ou laissées sécher.

L'exemple suivant sert à illustrer l'invention sans toutefois présenter un caractère limitatif.

### EXEMPLE

On prépare les compositions suivantes (les quantités sont exprimées en g% de matière active) :

**Composition 1**

| | |
|---|---|
| Disteardimonium hectorite (Bentone 38 VCG) | 3 |
| Octyldodécanol | 11,5 |
| Distéarate de glycol | 8 |
| Huile de vaseline | 64,5 |
| Carbonate de propylène | 1 |
| Laureth-2 | 1 |
| Polysorbate 21 | 11 |

**Composition 2**

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR, Aqualon) | 1,5 |
| Hexylène glycol | 3 |
| Dipropylène glycol | 3 |
| Ethanol | 8,25 |
| Propylèneglycol | 6,2 |
| Acide ascorbique | 0,25 |
| eau | Qsp 100 |

**Composition 3**

| | |
|---|---|
| Pentasodium pentetate | 0,15 |
| Peroxyde d'hydrogène (solution aqueuse à 50 %) | 12 |
| Stannate de sodium | 0,04 |
| Acide phosphorique | Qs pH 2.2 |
| Pyrophosphate tétrasodique | 0,03 |
| Huile de vaseline | 20 |
| Polycondensat tétraméthyl hexaméthylènediamine/ dichloro 1,3-propylène (solution aqueuse à 40% ; Hexadimethrine chloride) | 0,1 |
| Chlorure de polydiméthyl diallyl ammonium (solution aqueuse à 40 % non stabilisé, Polyquaternium-6) | 0,2 |
| Glycérine | 0,5 |
| Alcool cétylstéarylique (C₁₆/C₁₈ 30/70 - NAFOL 1618F) | 8 |
| Alcool cétylstéarylique oxyéthyléné (33 OE) | 3 |
| Amide d'acides de colza oxyethylene (4 OE) | 1,2 |
| Vitamine E : DL-α-tocophérol | 0,1 |
| eau | Qsp 100 |

### Mode d'application

Les trois compositions détaillées ci-dessus sont mélangées au moment de l'emploi dans les proportions suivantes :
* 10 g de la composition 1
* 4 g de la composition 2
* 16 g de la composition 3.

Le mélange résultant est ensuite appliqué sur des mèches de cheveux naturels à 90 % de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches châtain clair (évaluation visuelle).
* dans l'un, une première composition comprenant un ou plusieurs corps gras ; éventuellement un ou plusieurs colorants choisis parmi les colorants d'oxydation, les colorants directs, ou leurs mélanges ;
* dans l'autre, une deuxième composition comprenant un ou plusieurs agents oxydants ;
* le ou les polymères cationiques se trouvant dans la première et/ou dans la deuxième compositions ;
* le ou les agents alcalinisants optionnels se trouvant de préférence dans la première composition ;
* les compositions des deux compartiments étant destinées à être mélangées pour donner la composition selon l'invention, juste avant l'application sur les fibres kératiniques humaines, de telle sorte la composition résultant du mélange soit telle que définie précédemment.

L'invention concerne également un dispositif à deux compartiments comprenant dans l'un, une première composition comprenant un ou plusieurs corps gras, un ou plusieurs colorants d'oxydation, dans l'autre, une deuxième composition comprenant un ou plusieurs agents oxydants ; la première composition et/ou la deuxième composition comprenant un ou plusieurs polymères cationiques et éventuellement un ou plusieurs agents alcalinisants, ce ou ces derniers étant de préférence dans la première composition; les compositions des deux compartiments étant destinées à être mélangées pour donner la composition selon l'invention, juste avant l'application sur les fibres kératiniques humaines.

L'invention concerne de plus un dispositif à trois compartiments renfermant :
* dans l'un, une première composition comprenant un ou plusieurs corps gras ;
* dans un autre, une deuxième composition renfermant un ou plusieurs colorants choisis parmi les colorants d'oxydation, les colorants directs, ou leurs mélanges ; un ou plusieurs agents alcalinisants ; ou leurs mélanges ;
* et dans le dernier, une troisième composition comprenant un ou plusieurs agents oxydants ;
* le ou les polymères cationiques se trouvant dans la première, la deuxième et/ou la troisième compositions ;
* les compositions des trois compartiments étant destinées à être mélangées pour donner la composition selon l'invention, juste avant l'application sur les fibres kératiniques humaines, de telle sorte la composition résultant du mélange soit telle que définie précédemment.

Elle concerne de même un dispositif à trois compartiments comprenant dans l'un, une première composition comprenant un ou plusieurs corps gras, dans un autre, une deuxième composition un ou plusieurs colorants d'oxydation, et dans le dernier, une troisième composition comprenant un ou plusieurs agents oxydants ; la première, deuxième et/ou la troisième compositions comprenant un ou plusieurs polymères cationiques et éventuellement un ou plusieurs agents alcalinisants, ce ou ces derniers étant de préférence dans la première ou la seconde composition ; les compositions des trois compartiments étant destinées à être mélangées pour donner la composition selon l'invention, juste avant l'application sur les fibres kératiniques humaines.

L'exemple suivant sert à illustrer l'invention sans toutefois présenter un caractère limitatif.

### EXEMPLE

On prépare les compositions suivantes (les quantités sont exprimées en g% de matière active) :

**Composition 1**

| | |
|---|---|
| Disteardimonium hectorite (Bentone 38 VCG) | 3 |
| Octyldodécanol | 11,5 |
| Distéarate de glycol | 8 |
| Huile de vaseline | 64,5 |
| Carbonate de propylène | 1 |
| Laureth-2 | 1 |
| Polysorbate 21 | 11 |

**Composition 2**

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR, Aqualon) | 1,5 |
| Hexylène glycol | 3 |
| Dipropylène glycol | 3 |
| Ethanol | 8,25 |
| Propylèneglycol | 6,2 |
| Acide ascorbique | 0,25 |
| eau | Qsp 100 |

**Composition 3**

| | |
|---|---|
| Pentasodium pentetate | 0,15 |
| Peroxyde d'hydrogène (solution aqueuse à 50 %) | 12 |
| Stannate de sodium | 0,04 |
| Acide phosphorique | Qs pH 2.2 |
| Pyrophosphate tétrasodique | 0,03 |
| Huile de vaseline | 20 |
| Polycondensat tétraméthyl hexaméthylènediamine/ dichloro 1,3-propylène (solution aqueuse à 40% ; Hexadimethrine chloride) | 0,1 |
| Chlorure de polydiméthyl diallyl ammonium (solution aqueuse à 40 % non stabilisé, Polyquaternium-6) | 0,2 |
| Glycérine | 0,5 |
| Alcool cétylstéarylique (C₁₆/C₁₈ 30/70 - NAFOL 1618F) | 8 |
| Alcool cétylstéarylique oxyéthyléné (33 OE) | 3 |
| Amide d'acides de colza oxyethylene (4 OE) | 1,2 |
| Vitamine E : DL-α-tocophérol | 0,1 |
| eau | Qsp 100 |

### Mode d'application

Les trois compositions détaillées ci-dessus sont mélangées au moment de l'emploi dans les proportions suivantes :
* 10 g de la composition 1
* 4 g de la composition 2
* 16 g de la composition 3.

Le mélange résultant est ensuite appliqué sur des mèches de cheveux naturels à 90 % de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches châtain clair (évaluation visuelle).

## Revendications

1. Composition de coloration de fibres kératiniques humaines, **caractérisée en ce qu'**elle comprend, dans un milieu cosmétiquement acceptable :
(a) au moins 25 % en poids d'un ou plusieurs corps gras ;
(b) un ou plusieurs polymères cationiques ;
(c) un ou plusieurs colorants d'oxydation;
(d) un ou plusieurs agents oxydants ;
(e) un ou plusieurs agents alcalinisants choisis parmi les carbonates ou bicarbonates alcalins, les hydroxydes de sodium ou de potassium et les amines organiques dont le pKb à 25°C est inférieur à 12, et de préférence inférieur à 10, encore plus avantageusement inférieur à 6.

2. Composition selon la revendication précédente, **caractérisée en ce que** le ou les corps gras sont choisis parmi les alcanes inférieurs en C₆-C₁₆, les huiles non siliconées d'origine animale, végétale, minérale ou synthétique, les alcools gras, les acides gras, les esters d'acide gras et/ou d'alcool gras, les cires non siliconées, les silicones.

3. Composition selon l'une quelconque des revendications 1 ou 2, **caractérisée en ce que** le ou les corps gras sont choisis parmi les composés liquides ou pâteux et de préférence liquides à température ambiante et à pression atmosphérique.

4. Composition selon l'une quelconque des revendications précédentes dans laquelle le corps gras est choisi parmi les alcanes inférieurs en C₆-C₁₆, les huiles non siliconées d'origine végétale, minérale ou synthétique, les alcools gras, les acides gras, les esters d'acide gras et/ou d'alcool gras, ou leurs mélanges.

5. Composition selon l'une quelconque des revendications précédentes, **caractérisée en ce que** la teneur en corps gras varie de 25 à 80% en poids, de préférence de 25 et 65% en poids, encore plus particulièrement de 30 à 55% en poids, par rapport au poids de la composition.

6. Composition selon l'une quelconque des revendications précédentes, **caractérisée en ce que** le polymère cationique est choisi parmi les polymères suivants, seuls ou en mélanges :
**(1)** Les homopolymères ou copolymères dérivés d'esters ou d'amides acryliques ou méthacryliques et comportant au moins un des motifs de formules (I), (II), (III) ou (IV) suivantes: 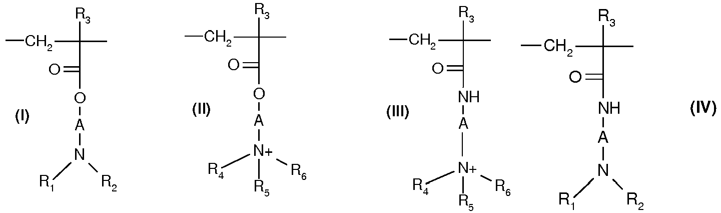 dans lesquelles :
R₃, identiques ou différents, désignent un atome d'hydrogène ou un radical CH₃ ;
A, identiques ou différents, représentent un groupe alkyle, linéaire ou ramifié, en C₁-C₆,
ou un groupe hydroxyalkyle en C₁-C₄ ;
R₄, R₅, R₆, identiques ou différents, représentent un groupe alkyle en C₁-C₁₈ ou un radical benzyle ;
R₁ et R₂, identiques ou différents, représentent hydrogène ou un groupe alkyle en C₁-C₆ ;
X désigne un anion dérivé d'un acide minéral ou organique ;
**(2)** Les dérivés d'éthers de cellulose comportant des groupements ammonium quaternaire ;
**(3)** Les dérivés de cellulose cationiques tels que les copolymères de cellulose ou les dérivés de cellulose greffés avec un monomère hydrosoluble d'ammonium quaternaire ;
**(4)** Les polysaccharides cationiques non cellulosiques ;
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères ;
**(6)** Les polyaminoamides solubles dans l'eau, préparés en particulier par polycondensation d'un composé acide avec une polyamine ;
**(7)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C₃-C₈ ;
**(8)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 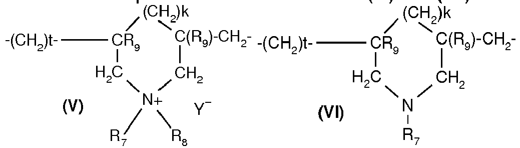 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle en C₁-C₈, un groupement hydroxyalkyle en C₁-C₅, un groupement amidoalkyle dont l'alkyle est en C₁-C₄ ; R₇ et R₈ peuvent encore désigner conjointement avec l'atome d'azote auquel ils sont rattachés, un groupement hétérocyclique ; Y⁻ est un anion organique ou minéral ;
**(9)** Les polymères de diammonium quaternaire contenant des motifs récurrents de formule : 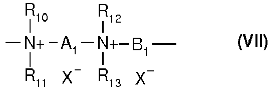 dans laquelle :
R₁₀, R₁₁, R₁₂ et R₁₃, identiques ou différents, représentent des radicaux aliphatiques, alicycliques, ou arylaliphatiques en C₁-C₂₀ ou des radicaux hydroxyalkylaliphatiques dont le radical alkyle est en C₁-C₄, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
A₁ et B₁ représentent des groupements polyméthyléniques en C₂-C₂₀, linéaires ou ramifiés, saturés ou non, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A₁, R₁₀ et R₁₂ peuvent former avec les deux atomes d'azote auxquels ils sont rattachés un cycle pipérazinique ;
en outre si A₁ désigne un radical alkylène ou hydroxyalkylène linéaire ou ramifié, saturé ou insaturé, B1 peut également désigner un groupement -(CH₂)ₙ-CO-D-OC-(CH₂)ₙ- dans lequel n est compris entre 1 et 100 et D désigne :
a) un reste de glycol de formule : -O-Z-O-, où Z représente -(CH₂-CH₂-O)ₓ-CH₂-CH₂- ; -[CH₂-CH(CH₃)-O)_{y-}CH₂-CH(CH₃)- où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire ;
c) un reste de diamine bis-primaire de formule : -NH-Y-NH-, où Y désigne un radical hydrocarboné linéaire ou ramifié, ou bien le radical -CH₂-CH₂-S-S-CH₂-CH₂- ;
d) un groupement uréylène de formule : -NH-CO-NH-.
**(10)** Les polymères de polyammonium quaternaire constitués de motifs récurrents de formule (IX) : 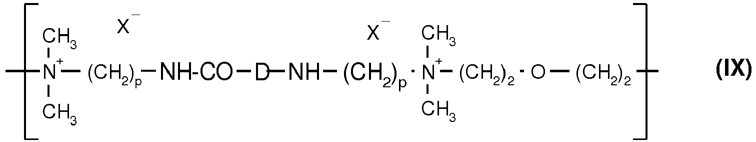 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
**(11)** Les polymères quaternaires de vinylpyrrolidone et de vinylimidazole ;
**(12)** Les homopolymères ou copolymères de vinylamide
**(13)** les dérivés de polyuréthanne cationique
**(14)** les hydrolysats de protéines cationiques, les polyalkylèneimines, les polymères contenant des motifs vinylpyridine ou vinylpyridinium, et les dérivés de la chitine.

7. Composition selon l'une quelconque des revendications précédentes, **caractérisée en ce que** le polymère cationique est choisi parmi les polymères dont la charge cationique est supérieure ou égale à 1 meq/g, de préférence supérieure ou égale à 2 meq/g.

8. Composition selon l'une quelconque des revendications précédentes, **caractérisée en ce que** le ou les polymères cationiques sont choisis parmi les polymères des familles (1), (2), (3), (4), (8), (9) et de préférence parmi les polymères des familles (8) et (9).

9. Composition selon l'une quelconque des revendications précédentes, **caractérisée** en ce la teneur en polymère(s) cationique(s) est comprise entre 0,005 à 5 % en poids, par rapport au poids de la composition.

10. Composition selon l'une quelconque des revendications précédentes, **caractérisé en ce qu'**elle comprend à titre de colorants d'oxydation, une ou plusieurs bases d'oxydation choisies parmi les paraphénylènediamines, les bis-phénylalkylènediamines, les para-aminophénols, les ortho-aminophénols, les bases hétérocycliques et leurs sels d'addition.

11. Composition la revendication précédente, caractérisé ce qu'elle comprend un ou plusieurs coupleurs choisis parmi les méta-phénylènediamines, les méta-aminophénols, les méta-diphénols, les coupleurs naphtaléniques, les coupleurs hétérocycliques ainsi que leurs sels d'addition.

12. Composition selon l'une quelconque des revendications précédentes, **caractérisé en ce qu'**elle comprend à titre de colorants directs, des colorants ioniques ou non ioniques, azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

13. Composition selon la revendication précédente, **caractérisée en ce que** l'amine organique est une alcanolamine, de préférence la monoéthanolamine.

14. Composition selon la revendication 13, **caractérisée en ce que** l'amine organique est choisie parmi les acides aminés basiques.

15. Procédé de coloration de fibres kératiniques humaines **caractérisé en ce que** l'on applique la composition selon l'une quelconque des revendications précédentes.

## Patentansprüche

1. Zusammensetzung zum Färben von menschlichen Keratinfasern, **dadurch gekennzeichnet, dass** sie in einem kosmetisch unbedenklichen Medium Folgendes umfasst:
(a) mindestens 25 Gew.-% einer oder mehrerer Fettsubstanzen;
(b) ein oder mehrere kationische Polymere;
(c) einen oder mehrere Oxidationsfarbstoffe;
(d) ein oder mehrere Oxidationsmittel;
(e) ein oder mehrere Alkalisierungsmittel, die aus Alkalicarbonaten oder -hydrogencarbonaten, Natrium- oder Kaliumhydroxid und organischen Aminen mit einem pKb-Wert bei 25 °C von weniger als 12, vorzugsweise weniger als 10 und noch vorteilhafter weniger als 6 ausgewählt sind.

2. Zusammensetzung nach dem vorhergehenden Anspruch, **dadurch gekennzeichnet, dass** die Fettsubstanz bzw. die Fettsubstanzen aus C₆-C₁₆-Niederalkanen, Nichtsilikonölen tierischer, pflanzlicher, mineralischer oder synthetischer Herkunft, Fettalkoholen, Fettsäuren, Fettsäure- und/oder Fettalkoholestern, Nichtsilikonwachsen und Silikonen ausgewählt ist bzw. sind.

3. Zusammensetzung nach Anspruch 1 oder 2, **dadurch gekennzeichnet, dass** die Fettsubstanz bzw. die Fettsubstanzen aus Verbindungen, die bei Umgebungstemperatur und Normaldruck flüssig oder pastös und vorzugsweise flüssig sind, ausgewählt ist bzw. sind.

4. Zusammensetzung nach einem der vorhergehenden Ansprüche, wobei die Fettsubstanz aus C₆-C₁₆-Niederalkanen, Nichtsilikonölen pflanzlicher, mineralischer oder synthetischer Herkunft, Fettalkoholen, Fettsäuren, Fettsäure- und/oder Fettalkoholestern oder Mischungen davon ausgewählt ist.

5. Zusammensetzung nach einem der vorhergehenden Ansprüche, **dadurch gekennzeichnet, dass** der Gehalt an Fettsubstanzen im Bereich von 25 bis 80 Gew.-%, vorzugsweise von 25 bis 65 Gew.-% und noch spezieller von 30 bis 55 Gew.-%, bezogen auf das Gewicht der Zusammensetzung, liegt.

6. Zusammensetzung nach einem der vorhergehenden Ansprüche, **dadurch gekennzeichnet, dass** das kationische Polymer aus den folgenden Polymeren alleine oder als Mischungen ausgewählt ist:
(1) Homopolymeren oder Copolymeren, die sich von Acrylsäure- oder Methacrylsäureestern oder -amiden ableiten und mindestens eine der Einheiten der nachstehenden Formeln (I), (II), (III) oder (IV) umfassen: 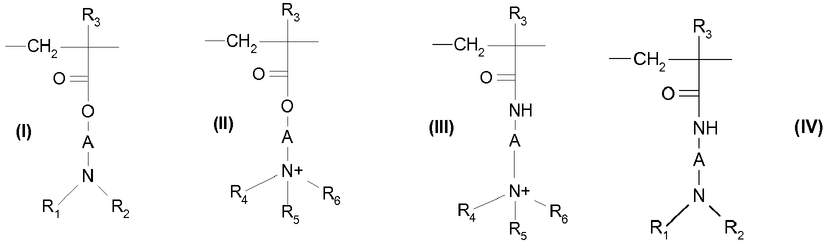 in denen:
die Variablen R₃, die gleich oder verschieden sind, für ein Wasserstoffatom oder einen CH₃-Rest stehen;
die Variablen A, die gleich oder verschieden sind, für eine lineare oder verzweigte C₁-C₆-Alkylgruppe oder eine C₁-C₄-Hydroxyalkylgruppe stehen;
R₄, R₅ und R₆, die gleich oder verschieden sind, für eine C₁-C₁₈-Alkylgruppe oder einen Benzylrest stehen;
R₁ und R₂, die gleich oder verschieden sind, für Wasserstoff oder eine C₁-C₆-Alkylgruppe stehen;
X für ein Anion steht, das sich von einer anorganischen oder organischen Säure ableitet;
(2) Celluloseetherderivaten mit quaternären Ammoniumgruppen;
(3) kationischen Cellulosederivaten wie Cellulosecopolymeren oder Cellulosederivaten, die mit einem wasserlöslichen quaternären Ammonium-Monomer gepfropft sind;
(4) nichtcellulosischen kationischen Polysacchariden;
(5) Polymeren, die aus Piperazinyleinheiten und zweiwertigen Alkylen- und Hydroxyalkylenresten mit geraden oder verzweigten Ketten, die gegebenenfalls durch Sauerstoff-, Schwefel- oder Stickstoffatome oder aromatische oder heterocyclische Ringe unterbrochen sind, bestehen, sowie den Oxidations- und/oder Quaternisierungsprodukten dieser Polymere;
(6) wasserlöslichen Polyaminoamiden, insbesondere hergestellt durch Polykondensation einer sauren Verbindung mit einem Polyamin;
(7) durch Reaktion eines Polyalkylenpolyamins mit zwei primären Amingruppen und mindestens einer sekundären Amingruppe mit einer aus Diglykolsäure und gesättigten aliphatischen C₃-C₈-Dicarbonsäuren ausgewählten Dicarbonsäure erhaltenen Polymeren;
(8) Cyclopolymeren von Alkyldiallylamin oder Dialkyldiallylammonium wie Homopolymeren oder Copolymeren, die als Hauptbestandteil der Kette Einheiten umfassen, die der Formel (V) oder (VI) entsprechen: 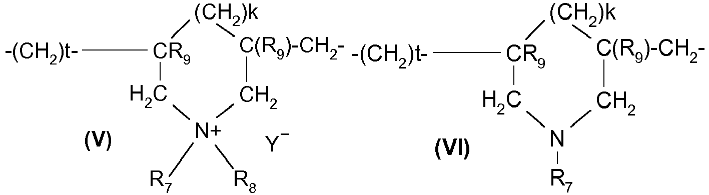 in denen k und t gleich 0 oder 1 sind, die Summe k + t gleich 1 ist; R₉ für ein Wasserstoffatom oder einen Methylrest steht; R₇ und R₈ unabhängig voneinander für eine C₁-C₈-Alkylgruppe, eine C₁-C₅-Hydroxyalkylgruppe oder eine Amidoalkylgruppe mit 1 bis 4 Kohlenstoffatomen im Alkylteil stehen; R₇ und R₈ auch zusammen mit dem Stickstoffatom, an das sie gebunden sind, für eine heterocyclische Gruppe stehen können; Y⁻ für ein organisches oder anorganisches Anion steht;
(9) quaternären Diammoniumpolymeren mit Wiederholungseinheiten der Formel: 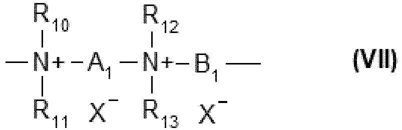 in der:
R₁₀, R₁₁, R₁₂ und R₁₃, die gleich oder verschieden sind, für aliphatische, alicyclische oder arylaliphatische C₁-C₂₀-Reste oder hydroxyalkylaliphatische Reste mit 1 bis 4 Kohlenstoffatomen im Alkylrest stehen oder auch R₁₀, R₁₁, R₁₂ und R₁₃ zusammen oder separat mit den Stickstoffatomen, an die sie gebunden sind, Heterocyclen bilden, die gegebenenfalls neben dem Stickstoff ein zweites Heteroatom enthalten, oder auch R₁₀, R₁₁, R₁₂ und R₁₃ für einen linearen oder verzweigten C₁-C₆-Alkylrest stehen, der durch eine Nitril-, Ester-, Acyl-, Amid- oder -CO-O-R₁₄-D- oder -CO-NH-R₁₄-D-Gruppe substituiert ist, wobei R₁₄ für ein Alkylen steht und D für eine quaternäre Ammoniumgruppe steht;
A₁ und B₁ für C₂-C₂₀-Polymethylengruppen stehen, die linear oder verzweigt und gesättigt oder ungesättigt sind und an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und
X⁻ für ein Anion, das sich von einer anorganischen oder organischen Säure ableitet, steht;
A₁, R₁₀ und R₁₂ mit den beiden Stickstoffatomen, an die sie gebunden sind, einen Piperazinring bilden können;
außerdem B₁ dann, wenn A₁ für einen linearen oder verzweigten, gesättigten oder ungesättigten Alkylen- oder Hydroxyalkylenrest steht, auch für eine-(CH₂)ₙ-CO-D-OC-(CH₂)ₙ-Gruppe stehen kann, worin n zwischen 1 und 100 liegt und D für:
a) einen Glykolrest der Formel: -O-Z-O-, wobei Z für -(CH₂-CH₂-O)ₓ-CH₂-CH₂- oder -[CH₂-CH(CH₃)-O]_{y}-CH₂-CH(CH₃) - steht, wobei x und y für eine ganze Zahl von 1 bis 4 stehen und einen definierten und einzigartigen Polymerisationsgrad wiedergeben oder für eine beliebige Zahl von 1 bis 4 stehen und einen durchschnittlichen Polymerisationsgrad wiedergeben;
b) einen bissekundären Diaminrest;
c) einen bisprimären Diaminrest der Formel: -NH-Y-NH-, wobei Y für einen linearen oder verzweigten Kohlenwasserstoffrest oder auch den Rest -CH₂-CH₂-S-S-CH₂-CH₂- steht;
d) eine Ureylengruppe der Formel: -NH-CO-NHsteht;
(10) quaternären Polyammoniumpolymeren, die aus Einheiten der Formel (IX) bestehen: 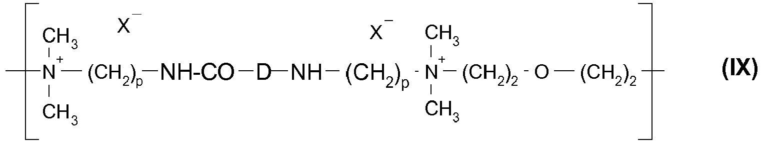 worin p für eine ganze Zahl im Bereich von ungefähr 1 bis 6 steht, D null sein kann oder für eine -(CH₂)ᵣ-CO-Gruppe, in der r eine ganze Zahl mit einem Wert von 4 oder 7 bedeutet, stehen kann und X⁻ für ein Anion steht;
(11) quaternären Vinylpyrrolidon- und Vinylimidazolpolymeren;
(12) Vinylamid-Homopolymeren und -Copolymeren;
(13) kationischen Polyurethanderivaten;
(14) kationischen Proteinhydrolysaten, Polyalkyleniminen, Polymeren mit Vinylpyridin- oder Vinylpyridinium-Einheiten und Derivaten wie Chitin.

7. Zusammensetzung nach einem der vorhergehenden Ansprüche, **dadurch gekennzeichnet, dass** das kationische Polymer aus Polymeren ausgewählt ist, deren kationische Ladung größer oder gleich 1 meq/g und vorzugsweise größer oder gleich 2 meq/g ist.

8. Zusammensetzung nach einem der vorhergehenden Ansprüche, **dadurch gekennzeichnet**, **dadurch gekennzeichnet, dass** das kationische Polymer bzw. die kationischen Polymere aus den Polymeren der Familien (1), (2), (3), (4), (8) und (9) und vorzugsweise aus den Polymeren der Familien (8) und (9) ausgewählt ist.

9. Zusammensetzung nach einem der vorhergehenden Ansprüche, dass der Gehalt an kationischem Polymer bzw. kationischen Polymeren zwischen 0,005 und 5 Gew.-%, bezogen auf das Gewicht der Zusammensetzung, liegt.

10. Zusammensetzung nach einem der vorhergehenden Ansprüche, **dadurch gekennzeichnet, dass** sie als Oxidationsfarbstoffe eine oder mehrere Oxidationsbasen, die aus para-Phenylendiaminen, Bisphenylalkylendiaminen, para-Aminophenolen, ortho-Aminophenolen, heterocyclischen Basen und den Additionssalzen davon ausgewählt sind, umfasst.

11. Zusammensetzung nach dem vorhergehenden Anspruch, **dadurch gekennzeichnet, dass** sie einen oder mehrere Kuppler, die aus meta-Phenylendiaminen, meta-Aminophenolen, meta-Diphenolen, Naphthalin-Kupplern, heterocyclischen Kupplern sowie den Additionssalzen davon ausgewählt sind, umfasst.

12. Zusammensetzung nach einem der vorhergehenden Ansprüche, **dadurch gekennzeichnet, dass** sie als Direktfarbstoffe ionische oder nichtionische AzoFarbstoffe, Methin-Farbstoffe, Carbonyl-Farbstoffe, Azin-Farbstoffe, Nitro(hetero)aryl-Farbstoffe, Tri(hetero)arylmethan-Farbstoffe, Porphyrine, Phthalocyanine und natürliche Direktfarbstoffe alleine oder als Mischungen umfasst.

13. Zusammensetzung nach dem vorhergehenden Anspruch, **dadurch gekennzeichnet, dass** es sich bei dem organischen Amin um ein Alkanolamin, vorzugsweise Monoethanolamin, handelt.

14. Zusammensetzung nach Anspruch 13, **dadurch gekennzeichnet, dass** das organische Amin aus basischen Aminosäuren ausgewählt ist.

15. Verfahren zum Färben von menschlichen Keratinfasern, **dadurch gekennzeichnet, dass** man die Zusammensetzung nach einem der vorhergehenden Ansprüche aufbringt.

## Claims

1. Composition for dyeing human keratin fibres, **characterized in that** it comprises, in a cosmetically acceptable medium:
(a) at least 25% by weight of one or more fatty substances;
(b) one or more cationic polymers;
(c) one or more oxidation dyes;
(d) one or more oxidizing agents;
(e) one or more basifying agents chosen from alkali metal carbonates or bicarbonates, sodium hydroxide, potassium hydroxide and organic amines whose pKb at 25°C is less than 12, preferably less than 10 and even more advantageously less than 6.

2. Composition according to the preceding claim, **characterized in that** the fatty substances are chosen from C₆-C₁₆ lower alkanes, non-silicone oils of animal, plant, mineral or synthetic origin, fatty alcohols, fatty acids, esters of a fatty acid and/or of a fatty alcohol, non-silicone waxes and silicones.

3. Composition according to either of Claims 1 and 2, **characterized in that** the fatty substance (s) is (are) chosen from compounds that are liquid or pasty and preferably liquid at room temperature and at atmospheric pressure.

4. Composition according to any one of the preceding claims, in which the fatty substance is chosen from C₆-C₁₆ lower alkanes, non-silicone oils of plant, mineral or synthetic origin, fatty alcohols, fatty acids, and esters of a fatty acid and/or of a fatty alcohol, or mixtures thereof.

5. Composition according to any one of the preceding claims, **characterized in that** the content of fatty substances ranges from 25% to 80% by weight, preferably from 25% to 65% by weight and even more particularly from 30% to 55% by weight relative to the weight of the composition.

6. Composition according to any one of the preceding claims, **characterized in that** the cationic polymer is chosen from the following polymers, alone or as mixtures:
**(1)** homopolymers or copolymers derived from acrylic or methacrylic esters or amides and comprising at least one of the units of formula (I), (II), (III) or (IV) below: 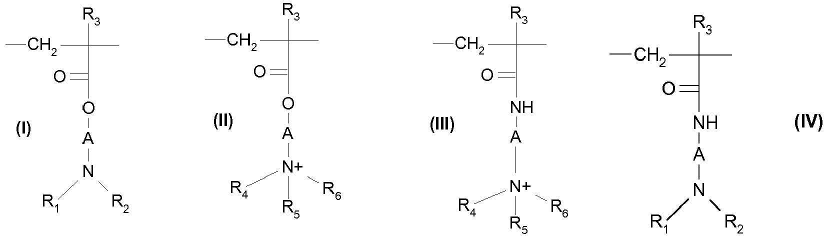 in which:
R₃, which may be identical or different, denote a hydrogen atom or a CH₃ radical;
A, which may be identical or different, represent a linear or branched C₁-C₆ alkyl group or a C₁-C₄ hydroxyalkyl group;
R₄, R₅ and R₆, which may be identical or different, represent a C₁-C₁₈ alkyl group or a benzyl radical;
R₁ and R₂, which may be identical or different, represent hydrogen or a C₁-C₆ alkyl group;
X denotes an anion derived from a mineral or organic acid;
**(2)** cellulose ether derivatives comprising quaternary ammonium groups;
**(3)** cationic cellulose derivatives such as cellulose copolymers or cellulose derivatives grafted with a water-soluble quaternary ammonium monomer;
**(4)** non-cellulose cationic polysaccharides;
**(5)** polymers consisting of piperazinyl units and of divalent alkylene or hydroxyalkylene radicals containing straight or branched chains, optionally interrupted by oxygen, sulfur or nitrogen atoms or by aromatic or heterocyclic rings, and also the oxidation and/or quaternization products of these polymers;
**(6)** water-soluble polyamino amides prepared in particular by polycondensation of an acidic compound with a polyamine;
**(7)** polymers obtained by reaction of a polyalkylene polyamine containing two primary amine groups and at least one secondary amine group with a dicarboxylic acid chosen from diglycolic acid and saturated C₃-C₈ aliphatic dicarboxylic acids;
**(8)** cyclopolymers of alkyldiallylamine or of dialkyldiallylammonium, such as the homopolymers or copolymers containing, as main constituent of the chain, units corresponding to formula (V) or (VI): 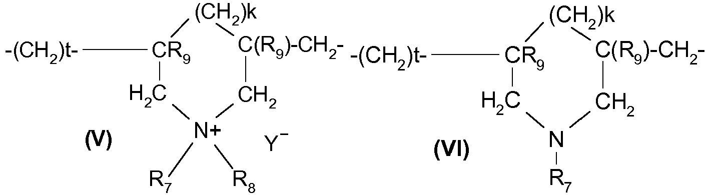 in which formulae k and t are equal to 0 or 1, the sum k + t being equal to 1; R₉ denotes a hydrogen atom or a methyl radical; R₇ and R₈, independently of each other, denote a C₁-C₈ alkyl group, a C₁-C₅ hydroxyalkyl group, an amidoalkyl group in which the alkyl is C₁-C₄; R₇ and R₈ can also denote, together with the nitrogen atom to which they are attached, a heterocyclic group; Y⁻ is an organic or mineral anion;
**(9)** the quaternary diammonium polymers containing repeating units of formula: 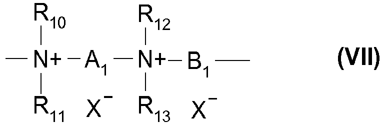 in which:
R₁₀, R₁₁, R₁₂ and R₁₃, which may be identical or different, represent C₁-C₂₀ aliphatic, alicyclic or arylaliphatic radicals or hydroxyalkylaliphatic radicals in which the alkyl radical is C₁-C₄, or alternatively R₁₀, R₁₁, R₁₂ and R₁₃, together or separately, constitute, with the nitrogen atoms to which they are attached, heterocycles optionally containing a second heteroatom other than nitrogen, or alternatively R₁₀, R₁₁, R₁₂ and R₁₃ represent a linear or branched C₁-C₆ alkyl radical substituted with a nitrile, ester, acyl or amide group or a group -CO-O-R₁₄-D or -CO-NH-R₁₄-D where R₁₄ is an alkylene and D is a quaternary ammonium group;
A₁ and B₁ represent C₂-C₂₀ polymethylene groups which may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
X⁻ denotes an anion derived from a mineral or organic acid;
A₁, R₁₀ and R₁₂ can form, with the two nitrogen atoms to which they are attached, a piperazine ring;
in addition, if A₁ denotes a linear or branched, saturated or unsaturated alkylene or hydroxyalkylene radical, B₁ can also denote a group -(CH₂)ₙ-CO-D-OC-(CH₂)ₙ- in which n is between 1 and 100, and D denotes:
a) a glycol residue of formula: -O-Z-O-, where Z represents -(CH₂-CH₂-O)ₓ-CH₂-CH₂-; -[CH₂-CH(CH₃)-O]_{y}-CH₂-CH(CH₃)-, where x and y denote an integer from 1 to 4, representing a defined and unique degree of polymerization or any number from 1 to 4 representing an average degree of polymerization;
b) a bis-secondary diamine residue;
c) a bis-primary diamine residue of formula: -NH-Y-NH-, where Y denotes a linear or branched hydrocarbon-based radical, or alternatively the radical -CH₂-CH₂-S-S-CH₂-CH₂-;
d) a ureylene group of formula: -NH-CO-NH-;
**(10)** polyquaternary ammonium polymers consisting of repeating units of formula (IX): 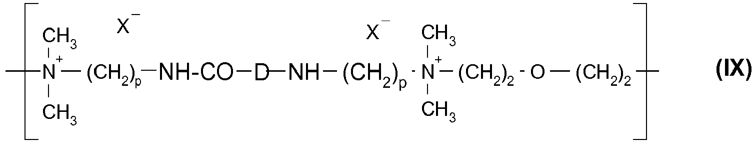 in which p denotes an integer ranging from 1 to 6 approximately, D may be zero or may represent a group -(CH₂)ᵣ-CO- in which r denotes a number equal to 4 or 7, and X⁻ is an anion;
**(11)** quaternary polymers of vinylpyrrolidone and of vinylimidazole;
**(12)** vinylamide homopolymers or copolymers;
**(13)** cationic polyurethane derivatives;
**(14)** cationic protein hydrolysates, polyalkylene-imines, polymers containing vinylpyridine or vinyl-pyridinium units, and chitin derivatives.

7. Composition according to any one of the preceding claims, **characterized in that** the cationic polymer is chosen from polymers whose cationic charge is greater than or equal to 1 meq./g and preferably greater than or equal to 2 meq./g.

8. Composition according to any one of the preceding claims, **characterized in that** the cationic polymer(s) is (are) chosen from polymers of families (1), (2), (3), (4), (8) and (9) and preferably from polymers of families (8) and (9).

9. Composition according to any one of the preceding claims, **characterized in that** the content of cationic polymer(s) is between 0.005% and 5% by weight relative to the weight of the composition.

10. Composition according to any one of the preceding claims, **characterized in that** it comprises, as oxidation dyes, one or more oxidation bases chosen from para-phenylenediamines, bis(phenyl)alkylenediamines, para-aminophenols, ortho-aminophenols and heterocyclic bases, and the addition salts thereof.

11. Composition according to the preceding claim, **characterized in that** it comprises one or more couplers chosen from meta-phenylenediamines, meta-aminophenols, meta-diphenols, naphthalene-based couplers and heterocyclic couplers, and also the addition salts thereof.

12. Composition according to any one of the preceding claims, **characterized in that** it comprises, as direct dyes, ionic or nonionic azo dyes; methine dyes; carbonyl dyes; azine dyes; nitro (hetero)aryl dyes; tri(hetero)arylmethane dyes; porphyrin dyes; phthalocyanine dyes; and natural direct dyes, alone or as mixtures.

13. Composition according to the preceding claim, **characterized in that** the organic amine is an alkanolamine, preferably monoethanolamine.

14. Composition according to Claim 13, **characterized in that** the organic amine is chosen from basic amino acids.

15. Process for dyeing human keratin fibres, **characterized in that** the composition according to any one of the preceding claims is applied.
